# EUROPÄISCHE PATENTSCHRIFT

(11) **EP 1 204 628 B1**
(45) Veröffentlichungstag und Bekanntmachung des Hinweises auf die Patenterteilung: **17.08.2005**
(21) Anmeldenummer: 00958412.9
(22) Anmeldetag: 10.08.2000
(51) Int. Cl.: C07C 51/573, C07C 63/16, B01D 3/14

(54) **VERFAHREN ZUR HERSTELLUNG VON SPEZIFIKATIONSGERECHTEM PHTHALSÄUREANHYDRID**
METHOD FOR PRODUCING PHTHALIC ANHYDRIDE ACCORDING TO SPECIFICATIONS
PROCEDE POUR PRODUIRE DE L'ANHYDRIDE PHTALIQUE CONFORME AUX SPECIFICATIONS

(30) Priorität: 20.08.1999 DE 19939629
(43) Veröffentlichungstag der Anmeldung: 15.05.2002
(73) Patentinhaber: BASF AKTIENGESELLSCHAFT, 67056 Ludwigshafen (DE)
(72) Erfinder: PESCHEL, Werner, D-67251 Freinsheim (DE); BESSLING, Bernd, D-67269 Grünstadt (DE); REUTER, Peter, D-68199 Mannheim (DE); LORZ, Peter, Michael, D-67157 Wachenheim (DE); ULRICH, Bernhard, D-67278 Bockenheim (DE); KNAB, Jean, Werner, D-67117 Limburgerhof (DE); KUMMER, Matthias, D-67273 Weisenheim (DE); RÜHL, Thomas, D-67227 Frankenthal (DE)
(86) Internationale Anmeldenummer: PCT/EP2000/007759
(87) Internationale Veröffentlichungsnummer: WO 2001/014308

(56) Entgegenhaltungen:
- WO-A-00/53561
- US-A- 3 420 750
- US-A- 4 008 255
- US-A- 4 547 578

## Beschreibung

Die vorliegende Erfindung betrifft ein Verfahren zur Herstellung von spezifikationsgerechtem Phthalsäureanhydrid durch destillative Reinigung von rohem Phthalsäureanhydrid.

Phthalsäureanhydrid ist eine bedeutsame Grundchemikalie der chemischen Industrie. Es dient zu einem beträchtlichen Teil als Ausgangsstoff für Dialkylphthalate, die in großen Mengen als Weichmacher für Kunststoffe wie PVC zum Einsatz kommen. Rohes Phthalsäureanhydrid wird in der Technik aus Naphthalin und/oder o-Xylol durch katalytische Oxidation in der Gasphase hergestellt. Vorzugsweise setzt man ein rohes Phthalsäureanhydrid ein, das auf diesem Wege aus o-Xylol hergestellt wurde. Die Austräge dieser Verfahren weisen, bezogen auf ihr Gesamtgewicht, üblicherweise mehr als 99,5 Gew.-% Phthalsäureanhydrid auf. Das Phthalsäureanhydrid wird meist in flüssiger Form oder als Feststoff an Abscheidern isoliert und danach üblicherweise destillativ gereinigt. Dazu wird es flüssig oder nach Verdampfen der Destillationskolonne zugeführt.

In Abhängigkeit vom gewählten Herstellverfahren und dabei besonders von den Ausgangsstoffen und den Katalysatoren enthält das Produkt ein jeweils spezifisches Spektrum von Verunreinigungen und Nebenprodukten (vgl. z.B.: H. Suter: "Wissenschaftliche Forschungsberichte: II. Anwendungstechnik und angewandte Wissenschaft", Dr. Dietrich Steinkopff Verlag, Darmstadt, 1972, Seite 39 ff.; im Folgenden kurz "Suter" genannt).

Am Markt wird eine Phthalsäureanhydrid-Qualität mit folgenden Spezifikationsgrenzen erwartet:
Erstarrungspunkt (°C) min. 130,8
Massenanteile (Gew.-%):
   - PSA min. 99,8
   - MSA max. 0,05
   - Benzoesäure max. 0,10 bzw.
      max. 0,01 bei Lebensmittelqualität
   - Phthalsäure max. 0,1
Schmelzfarbzahl (Hazen) max. 20
Hitzefarbzahl (Hazen) max. 40

Weil für die meisten Verwendungszwecke ein Phthalsäureanhydrid ohne verfärbende Verunreinigungen benötigt wird, kommt dabei der Charakterisierung durch die sogenannten Farbzahlen - vorrangig der Schmelzfarbzahl und der Hitzefarbzahl - besondere Bedeutung zu. Farbliche Veränderungen des Phthalsäureanhydrids unter thermischer Belastung sind deswegen von praktischer Bedeutung, weil Phthalsäureanhydrid normalerweise im geschmolzenen Zustand - etwa bei 160°C - gelagerz und transportiert wird.

In der Technik hat sich über die lange Zeit, in der Phthalsäureanhydrid bereits im industriellen Maßstab hergestellt wird, die Abtrennung dieser Nebenprodukte mittels Destillation durchgesetzt (vgl. z .B. : "Ullmann's Encyclopedia of Industrial Chemistry", 5. Edition, Vol. A20. VCH Verlagsgesellschaft mbH, Weinheim, 1992, Seiten 181 bis 189; in Folgenden kurz "Ullmann" genannt; Kirk-Othmer "Encyclopedia of Chemical Technology", 4. Edition, Vol. 18, John wiley & Sons, New York, 1996, Seiten 997 bis 1006, im Folgenden kurz "Kirk-Othmer" genannt). Niedrig siedende und/oder azeotrop destillierende Verunreinigungen, teilweise mit intensiver Eigenfarbe, bereiten dem Fachmann trotz vergleichsweise geringer Anteile dabei große Mühe. Praktisch geht man in der Technik daher so vor, dass man das rohe Phthalsäureanhydrid einer kombinierten Reinigung aus thermischer Vorbehandlung und Destillation unterwirft.

Die thermische Vorbehandlung wird bei Temperaturen von 220 - 280°C und einer Verweilzeit im Reaktor von mehreren Stunden bis zu einem Tag durchgeführt. Die übrigen Randbedingungen der thermischen Vorbehandlung richten sich im Allgemeinen nach Herkunft und damit Zusammensetzung des rohen Phthalsäureanhydrids. Sie dient verschiedenen, dem Fachmann geläufigen Zwecken, z.B. wird das Nebenprodukt Phthalsäure offenbar zum Wertprodukt Phthalsäureanhydrid dehydratisiert, was auch bei geringen Gehalten an Phthalsäure bei den großen technisch hergestellten Mengen an Phthalsäureanhydrid von erheblicher wirtschaftlicher Bedeutung ist. Das dabei gebildete oder sonstiges Wasser wird bei der thermischen Vorbehandlung entfernt, weil es bei der anschließenden Destillation stören kann. Weiterhin verharzen durch die thermische Vorbehandlung bestimmte Nebenprodukte der Synthesereaktion, was die folgende destillative Reinigung des Phthalsäureanhydrids erleichtert (vgl. z.B.: "Suter", Seiten 41 - 45). Es können bei der thermischen Vorbehandlung auch noch bestimmte chemische Stoffe zugesetzt werden, um das Spektrum der Nebenprodukte vor dem Destillationsschritt gezielt zu verändern.

Die Destillation - vor allem ihre unter wirtschaftlichen Aspekten häufig besonders interessante kontinuierliche Durchführung - erfolgt üblicherweise mittels zweier Kolonnen, um ein hinreichend reines Phthalsäureanhydrid zu erhalten. Im ersten Schritt werden dabei in der Regel die Leichtsieder (beispielsweise Benzoesäure, Maleinsäureanhydrid, Citraconsäureanhydrid), also Stoffe mit einem Siedepunkt unterhalb des Siedepunktes des Phthalsäureanhydrids abgetrennt; in einem zweiten Schritt destilliert man dann Phthalsäureanhydrid von den Schwersiedern (beispielsweise Phthalsäure, bestimmte farbgebende Komponenten, Kondensationsprodukte aus Inhaltsstoffen des rohen Phthalsäureanhydrids), also Stoffen mit höheren Siedepunkten als dem von Phthalsäureanhydrid bzw. von undestillierbaren Bestandteilen ab.

In "Suter" (dort auf Seite 45) wird bereits auf eine einstufige kontinuierliche Destillation von Phthalsäureanhydrid verwiesen (Ruhröl, Europa-Chemie 21, 7 (1965)), wobei keine weiteren Einzelheiten genannt werden.

US-A 4,008,255 betrifft ein Verfahren zur Gewinnung von Phthalsäureanhydrid, bei dem das rohe, gasförmige Phthalsäureanhydrid in einem ersten Reinigungsschritt in einer Absorptionskolonne durch Paraffin oder hochsiedende Kohlenwasserstoffe absorbiert und das nach Kühlung abgeschiedene feste Phthalsäureanhydrid nach mechanischer Abtrennung vom flüssigen Absorptionsmittel in einem zweiten Reinigungsschritt einer Destillation in einer Destillationskolonne mit Seitenabzug unterzogen wird.

US-A 4,547,578 beschreibt ein Verfahren zur Abtrennung von u.a. Naphthochinon-Verunreinigungen in rohem Phthalsäureanhydrid, in dem dieses bei erhöhter Temperatur mit einem polymeren 1,3-Dien-Öl, beispielsweise Polybutadien oder Polyisopren, mit einem Molekulargewicht von 800 bis 5000 behandelt wird.

US-A 3,420,750 lehrt eine zweistufige Destillation zur kontinuierlichen Reinigung von rohem Phthalsäureanhydrid.

Zusammenfassend lässt sich feststellen, dass die Reinigung von rohem Phthalsäureanhydrid ein sehr aufwendiger Prozeß hinsichtlich der Anlagen- und Betriebskosten ist, vor allem wenn es um die für viele praktische Anwendungen erforderliche Produktqualität geht. Der Schritt der thermischen Vorbehandlung wurde trotz des damit verbundenen erhöhten Aufwandes in der Technik dennoch bis heute beibehalten.

Der vorliegenden Erfindung lag daher die Aufgabe zugrunde, ein technisch einfaches und damit wirtschaftliches Verfahren zu finden, mit dem rohes Phthalsäureanhydrid so gereinigt werden kann, dass die am Markt verlangten Spezifikationen erreicht werden.

Demgemäß wurde ein Verfahren zur Herstellung von spezifikationsgerechtem Phthalsäureanhydrid durch destillative Reinigung von rohem Phthalsäureanhydrid gefunden, bei dem man rohes Phthalsäureanhydrid einer Destillationskolonne, die bei vermindertem Druck betrieben wird, zuführt, die Leichtsieder am Kopf oder in der Nähe des Kopfes der Destillationskolonne und das spezifikationsgerechte Phthalsäureanhydrid über einen Seitenabzug aus der Kolonne entnimmt.

Mit dem erfindungsgemäßen Verfahren ist es möglich, ohne die in der Technik üblich gewordene thermische Vorbehandlung ein Phthalsäureanhydrid hoher Reinheit zu erhalten, das die allgemein bekannten Spezifikationen von reinem Phthalsäureanhydrid erfüllt oder diese insbesondere bei den Farbzahlen sogar übertrifft: Man erhält in der Regel ein Phthalsäureanhydrid mit einer Schmelzfarbzahl von weniger als 10 APHA und einer Hitzefarbzahl von weniger als 20 APHA.

Geeignete Destillationskolonnen (im Folgenden auch kurz "Kolonnen" genannt) im Sinne der vorliegenden Erfindung sind Bodenkolonnen, Füllkörperkolonnen und Packungskolonnen sowie Kolonnen, in denen die technischen Merkmale dieser Kolonnentypen kombiniert wurden. Bevorzugt verwendet man Bodenkolonnen. Es sind bei den genannten Kolonnentypen übliche Einbauten wie handelsübliche Böden, Füllkörper oder Packungen, beispielsweise Glockenböden, Tunnelböden, Ventilböden, Siebböden, Dualflowböden und Gitterböden, Pall-Ringe®, Berl®-Sattelkörper, Netzdrahtringe, Raschig-Ringe®, Intalox®-Sättel, Interpak®-Füllkörper und Intos® aber auch geordnete Packungen, wie beispielsweise Sulzer-Mellapak®, Sulzer-Optiflow®, Kühni-Rombopak® und Montz-Pak® sowie Gewebepackungen verwendbar. Im Bereich unterhalb des Zulaufes der Kolonne werden vorzugsweise Einbauten gewählt, die auch feststofftauglich sind, besonders Dual-Flow-Böden. Geeignet sind hierfür in der Regel Böden und Füllkörper der oben genannten Bauarten.

Die Kolonne ist in der Regel mit einem Sumpfverdampfer sowie mit einem Kondensator am Kolonnenkopf ausgestattet.

Der Durchmesser der Kolonne richtet sich nach den jeweils angestrebten Durchsätzen und kann nach den gängigen Regeln der Technik vom Fachmann problemlos ermittelt werden.

Die Höhe der Kolonne sowie die Lagen von Zulauf und Seitenabzug können mit dem Konzept der theoretischen Trennstufen in Verbindung mit den gewählten Einbauten ermittelt werden.

Als eine theoretische Trennstufe wird diejenige Kolonneneinheit definiert, die ein Anreichern der leichtflüchtigen Komponente entsprechend dem thermodynamischen Gleichgewicht bewirkt, vorausgesetzt, dass ideale Durchmischung vorliegt, beide Phasen im Gleichgewicht stehen und kein Mitreißen von Flüssigkeitstropfen erfolgt (vgl. Vauck, Müller: Grundoperationen chemischer Verfahrenstechnik, VCH Verlagsgesellschaft mbH, Weinheim, 1988).

Im Allgemeinen ist die erfindungsgemäße Kolonne in drei Abschnitte unterteilt, die durch die Lagen von Zulauf, Seitenabzug, Kopf und Sumpf festgelegt werden. Die Zahl der theoretischen Trennstufen für die beiden oberen Abschnitte und der Rücklauf der Kolonne werden nach üblichen verfahrenstechnischen Überlegungen in Abhängigkeit vom Leichtsiederanteil im rohen Phthalsäureanhydrid und dem gewünschten Restgehalt an Leichtsiedern im gereinigten Phthalsäureanhydrid festgelegt. Die Zahl der theoretischen Trennstufen für den unteren Abschnitt der erfindungsgemäßen Kolonne beträgt im Allgemeinen 1 bis 10, vorzugsweise 2 bis 8 und vor allem 3 bis 7.

Besonders eignet sich das erfindungsgemäße Verfahren für rohes Phthalsäureanhydrid, wie es durch katalytische Gasphasenoxidation von o-Xylol erhalten wird und welches vorzugsweise mehr als 95 und insbesondere mehr als 98 Gew.-% Phthalsäureanhydrid aufweist.

Die Kolonne wird im Allgemeinen bei einem absoluten Druck am Kolonnenkopf von 0,05 bis 0,5, bevorzugt 0,1 bis 0,3, besonders bevorzugt bei 0,15 bis 0,25 und ganz besonders bevorzugt um 0,2 bar betrieben. Die Temperaturen in der Kolonne liegen im Allgemeinen bei etwa 160 bis 230°C am Kolonnenkopf und bei 180 bis 270°C am Kolonnensumpf. Die Temperatur am Seitenabzug liegt im Allgemeinen bei 170 bis 260, vorzugsweise bei 200 bis 240°C.

Die Destillation kann diskontinuierlich und, vorzugsweise, kontinuierlich durchgeführt werden. Das rohe Phthalsäureanhydrid wird der Kolonne vorzugsweise flüssig oder gasförmig, insbesondere flüssig zugeführt. Die Entnahme in flüssiger Form erfolgt normalerweise oberhalb des Zulaufs der Kolonne, die Entnahme im gasförmigen Zustand, die bevorzugt ist, erfolgt normalerweise unterhalb des Zulaufs.

In einer bevorzugten Ausführungsform des erfindungsgemäßen Verfahrens bei Verwendung von Bodenkolonnen können technische Einrichtungen wie Tropfenabscheider an dem Seitenabzug innerhalb oder außerhalb der Kolonne angebracht werden.

Als besonders vorteilhaft hat es sich erwiesen, eine Bodenkolonne zu verwenden, in der der Boden unterhalb und der Boden oberhalb der Stelle, an der sich der Seitenabzug befindet, zueinander einen gegenüber dem üblichen Abstand der Böden in der Kolonne vergrößerten, vorzugsweise einen bis zu fünffachen und insbesondere einen zwei- bis dreifachen Abstand haben, wobei sich der Seitenabzug vorzugsweise näher am oberen der beiden Böden befindet.

In einer bevorzugten Verfahrensvariante sind die Kolonnenböden dergestalt angeordnet, daß der unterste Boden gegenüber der üblichen Position in der Kolonne um das bis zu 6-fache, bevorzugt 2- bis 4-fache, besonders bevorzugt um das 3-fache des Abstandes der Böden in der Kolonne zueinander höher angeordnet ist. In dieser Verfahrensvariante werden die Anlageverfügbarkeit ung die Qualitätssicherheit weiter verbessert.

Üblicherweise wird der unterste Boden einer Kolonne mit gewissem Abstand gegenüber dem Brüdenrohr des Sumpfverdampfers angeordnet. Es wurde gefunden, daß bei in üblicher Position eingebauten Kolonnenböden auf den untersten 6, insbesondere aber auf den untersten 3 Böden beim Betrieb zur Durchführung des erfindungsgemäßen Verfahrens starke Verkrustungen auftraten, die nur mühsam mechanisch abgelöst werden konnten. Starke Verkrustungen traten auch in den Ablaufschächten der genannten untersten Böden auf, wie auch starke Beläge sowie lose koksartige Brocken unterschiedlicher Größen im Brüdenrohr des Sumpfverdampfers. Es wurde ein starker Druckabfall über die untersten stark verschmutzten Böden, von bis zu 130 mbar, festgestellt, der mit steigender Betriebsdauer zunahm. Überraschend wurde gefunden, daß die oben beschriebenen starken Verschmutzungen und die damit in Zusammenhang stehenden auftretenden Probleme beim Betrieb der Kolonne weiterhin vermieden werden konnten, indem, wie oben dargestellt, der unterste Boden entsprechend hoch angeordnet wird.

Eine weitere Verbesserung des erfindungsgemäßen Verfahrens, insbesondere bezüglich der Verschmutzungsproblematik, kann erreicht werden, indem in einer bevorzugten Ausführungsvariante zumindest die untersten 1 bis 6, bevorzugt 2 bis 4, besonders bevorzugt 3 Böden als Dual-Flow-Böden ausgebildet werden. Weiter bevorzugt können zumindest die Böden unterhalb des Seitenabzugs als Dual-Flow-Böden ausgebildet werden. Es ist jedoch auch möglich, alle Böden unterhalb der Zuführung des rohen Phthalsäureanhydrids als Dual-Flow-Böden auszubilden.

Eine weitere Verbesserung des erfindungsgemäßen Verfahrens sieht vor, daß der Sumpfverdampfer der Kolonne als Fallfilmverdampfer ausgestaltet ist. Als Sumpfverdampfer für Kolonnen zur Destillation von Phthalsäureanhydrid werden in der Regel sogenannte Zwangsumlaufentspannungsverdampfer verwendet. Fallfilmverdampfer wird der Fachmann dagegen nicht einsetzen, wenn die zu behandelnde Flüssigkeit (wie vorliegend) Feststoffe enthält, wegen der Gefahr, daß sich die Rohre zusetzen könnten. Überraschend wurde gefunden, daß dieses bekannte Problem nicht auftrat und daß der Apparat keine Ablagerungen aufwies. In den erkalteten Rohren wurden lediglich graue nadelförmige Kristalle gefunden, im wesentlichen von kondensiertem Phthalsäureanhydrid, die sich leicht von den Rohrwänden abstoßen ließen. Fallfilmverdampfer sind in der Verfahrenstechnik allgemein bekannt. Sie haben die Vorteile einer geringeren mittleren Verweilzeit der Flüssigkeit im Verdampfungsbereich mit der Folge einer schonenderen Verdampfung gegenüber Zwangsumlaufentspannungsverdampfern. Durch die schonendere Behandlung konnte die Neigung zur Feststoffbildung vermindert werden, die geringen Verweilzeiten führten zu einer Reduzierung von unerwünschten Nebenreaktionen und somit zu einer verbesserten Ausbeute im Sumpf, und die Betriebskosten konnten gesenkt werden.

Das spezifikationsgerechte Phthalsäureanhydrid wird üblicherweise unmittelbar nach der Entnahme aus der Kolonne gekühlt und als Flüssigkeit oder, nach dem Erstarren, als Festkörper erhalten. Ein höherer Reinheitsgrad kann gewünschtenfalls erreicht werden, indem man das Phthalsäureanhydrid beispielsweise über eine Seitenkolonne feindestilliert oder ein Trennblech achsial über einen bestimmten Bereich in der Kolonne (sog. Petlyuk-Verschaltung) anbringt. Auch ein Umkristallieren kommt hier in Betracht.

Über den Seitenabzug wird das spezifikationsgerechte Phthalsäureanhydrid normalerweise in einer Menge von mindestens 97 Gew.-% bezogen auf das Gewicht des Zulaufs abgenommen. Bei kontinuierlichem Betrieb der Kolonne liegt die entnommene Menge vorzugsweise bei mindestens 90, besonders bevorzugt bei mindestens 95 Gew.-%, bezogen auf das Gewicht des Zulaufs.

Die Wiedergewinnungsrate an Phthalsäureanhydrid bezogen auf den Gehalt im Zustrom zur Kolonne liegt in der Regel bei 98 % und höher.

Die bei der Destillation anfallenden Leichtsieder sowie Hochsieder werden üblicherweise in die Verbrennung gegeben.

Gewünschtenfalls können dem rohen Phthalsäureanhydrid vor oder bei der Destillation zusätzlich ein oder mehrere solcher chemischen Stoffe zugesetzt werden, die nach allgemeinem Fachwissen den Anteil unerwünschter Begleitstoffe, insbesondere solche, die die Stofftrennung bei der Destillation erschweren, absenken können. Beispielhaft seien genannt: Alkalihydroxid, etwa zur Verminderung des Phthalid-Gehaltes (vgl. US-A 4,165,324), Natriumhydrogencarbonat, Schwefelsäure, Borsäure oder Mischungen von Schwefelsäure und Borsäure. Die Verwendung derartiger Zusätze ist nicht Teil der Erfindung.

Es versteht sich, dass das erfindungsgemäße Verfahren für individuelle Trennprobleme noch optimal angepasst werden kann. Diese Anpassung kann der Fachmann routinemäßig vornehmen, wenn er der offenbarten Lehre folgt.

Die Reinheit des so erhaltenen Phthalsäureanhydrids läßt sich anhand allgemein bekannter analytischer Methoden wie der Gaschromatographie, der UV-Spektroskopie und der Säure-Base-Titration ermitteln. Insbesondere die Schmelzfarbzahl (APHA/Hazen-Farbskala, vgl. W. Liekmeier, D. Thybusch: Charakterisierung der Farbe von klaren Flüssigkeiten, Editor: Bodenseewerk Perkin-Elmer GmbH, Überlingen, 1991) wird im Allgemeinen so ermittelt, dass man die Farbzahl von Phthalsäureanhydrid unmittelbar nach der Probenahme bei einer Temperatur von 160°C bestimmt. Weiterhin wird die Hitzefarbzahl im Allgemeinen so ermittelt, dass man das Phthalsäureanhydrid 90 Minuten lang bei 250°C hält und dann die Farbzahl misst.

### Beispiel 1

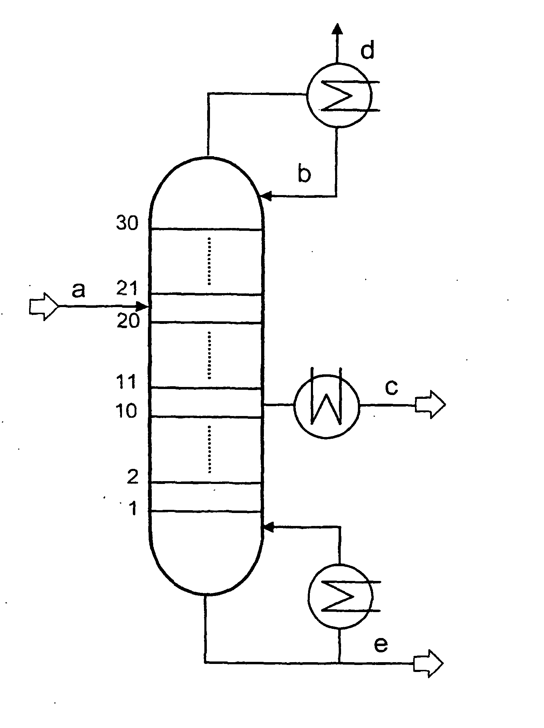

Es kam eine Bodenkolonne gemäß der schematischen Abbildung 1 zum Einsatz. Die Kolonne wies 30 Glockenböden (überschlagsmäßig 18 theoretische Trennstufen) auf und hatte einen Durchmesser von 50 mm. Der Seitenabzug befand sich zwischen dem 10. und dem 11. Boden über dem Sumpf (überschlagsmäßig im Bereich zwischen der fünften und der sechsten theoretischen Trennstufe), der Zulauf befand sich zwischen dem 20. und dem 21. Boden über dem Sumpf (überschlagsmäßig im Bereich der elften theoretischen Trennstufe). In Abbildung 1 sind noch der 1. und der 2. Boden eingezeichnet, die übrigen Böden sind durch senkrechte unterbrochene Linien angedeutet.

Es kam ein rohes Phthalsäureanhydrid zur Destillation, das durch Gasphasenoxidation von o-Xylol an einem Festbett in Gegenwart eines Katalysators, bestehend aus einem Trägerkern und den darauf schalenförmig aufgebrachten katalytisch wirksamen Metalloxiden Cäsiumoxid (berechnet als 0,4 Gew.-% Cäsium), Vanadiumoxid (4 Gew.-%) und Titandioxid (95,6 Gew.-%), hergestellt worden war (vgl. die ältere deutsche Patentanmeldung mit dem Aktenzeichen 198 24 532). Die Beladung im Reaktor betrug 86 g o-Xylol pro Nm³ Luft. Die Reaktortemperatur lag zwischen 350 und 450°C.

Das so erhaltene rohe Phthalsäureanhydrid hatte folgende Zusammensetzung:

| | |
|---|---|
| 99,24 Gew.-% | Phthalsäureanhydrid |
| 0,2 Gew.-% | Benzoesäure |
| 200 Gew.-ppm | Maleinsäureanhydrid |
| 20 Gew.-ppm | Citraconsäureanhydrid |
| 0,5 Gew.-% | Phthalsäure |

und der Rest zu 100 Gew.-% sonstige Stoffe.

1000 g dieses rohen Phthalsäureanhydrids wurden der Kolonne stündlich kontinuierlich zugeführt (a). Bei einem Rücklauf von 600 g (b), einem absoluten Druck von 0,2 bar am Kolonnenkopf, einer Temperatur von 200°C am Kolonnenkopf und 228°C am Kolonnensumpf wurden in der gleichen Zeit 980 g gereinigtes Phthalsäureanhydrid über den Seitenabzug bei 224°C entnommen, kondensiert und isoliert (c). Der Kopfabzug über (d) wurde dabei in eine Kühlfalle kondensiert und betrug ca. 5 g; der Sumpfabzug über (e) betrug ca. 15 g und enthielt die Schwersieder und nichtdestillierbaren Anteile. Die Analyse des über den Seitenabzug bei (c) isolierten Phthalsäureanhydrids ergab folgende Zusammensetzung:

| | |
|---|---|
| 99,97 Gew.-% | Phthalsäureanhydrid |
| 30 Gew.-ppm | Benzoesäure |
| < 10 Gew.-ppm | Maleinsäureanhydrid |
| < 10 Gew.-ppm | Citraconsäureanhydrid |
| 0,025 Gew.-% | Phthalsäure |

und der Rest zu 100 Gew.-% sonstige Stoffe.

Die Schmelzfarbzahl wurde unmittelbar nach der Entnahme ermittelt und betrug 5-10 APHA. Die Hitzefarbzahl wurde wie folgt ermittelt: Eine Probe Phthalsäureanhydrid wurde in einem Wärmeschrank 1,5 Stunden bei einer Temperatur von 250°C getempert. Anschließend wurde die Farbzahl =u 10-20 APHA gemessen.

### Beispiel 2

### Ausgehend von den nachfolgenden Vorgaben bezüglich Rohware und Reinprodukt:

0,3 Gew.-% Benzoesäure im durch das erfindungsgemäße destillative Verfahren zu reinigenden Phthalsäureanhydrid und 10 ppm Benzoesäure im Reinprodukt mit im übrigen unveränderter Zusammensetzung des rohen Phthalsäureanhydrids gegenüber Beispiel 1 wurde die Konfiguration der einzusetzenden Bodenkolonne in folgender Weise geändert:
- Anzahl der Böden zwischen gasförmigem Seitenabzug (Strom c) und Kolonnenkopf: entsprechend 19 theoretischen Trennstufen,
- Anzahl der Böden zwischen Zulauf (Strom a) und Kolonnenkopf: entsprechend 8 theoretischen Trennstufen,
- Anzahl der Böden zwischen Kolonnensumpf und gasförmigen Seitenabzug (Strom c): 10 Dual-Flow-Böden,
- absoluter Druck am Kolonnenkopf ca. 70 mbar,
- Temperatur im Kolonnensumpf: 230 bis 240°C,
- Bauform des Sumpfverdampfers: Fallfilmverdampfer,
- Abstand zwischen Seitenabzug (Strom c) und dem hierzu nächsten, darunter liegenden Boden; das 3-fache des üblichen Bodenabstandes in der Kolonne und
- Abstand Brüdenrohr des Sumpfverdampfers zum ersten darüber angeordneten Boden: das 3-fache des üblichen Bodenabstandes in der Kolonne zusätzlich zu dem üblichen Abstand des untersten Bodens gegenüber dem Brüdenrohr des Sumpfverdampfers.

## Patentansprüche

1. Verfahren zur Herstellung von spezifikationsgerechtem Phthalsäureanhydrid durch destillative Reinigung von rohem Phthalsäureanhydrid, **dadurch gekennzeichnet, dass** man rohes Phthalsäureanhydrid einer Destillationskolonne, die bei vermindertem Druck betrieben wird, zuführt, die Leichtsieder am Kopf oder in der Nähe des Kopfes der Destillationskolonne und das spezifikationsgerechte Phthalsäureanhydrid über einen Seitenabzug aus der Kolonne entnimmt.

2. Verfahren nach Anspruch 1, **dadurch gekennzeichnet, dass** man die Destillation bei einem Druck am Kolonnenkopf von 0,05 bis 0,5 bar durchführt.

3. Verfahren nach Anspruch 1 oder 2, **dadurch gekennzeichnet, dass** man die Destillation in einer Bodenkolonne durchführt.

4. Verfahren nach Anspruch 3, **dadurch gekennzeichnet, dass** der Boden unterhalb und der Boden oberhalb der Stelle, an der sich der Seitenabzug befindet, zueinander einen gegenüber dem üblichen Abstand der Böden in der Kolonne auf das bis zu Fünffache vergrößerten Abstand haben.

5. Verfahren nach Anspruch 4, **dadurch gekennzeichnet, dass** sich der Seitenabzug näher am oberen der beiden Böden befindet.

6. Verfahren nach den Ansprüchen 1 bis 5, **dadurch gekennzeichnet, dass** man das spezifikationsgerechte Phthalsäureanhydrid der Kolonne gasförmig über einen Seitenabzug entnimmt.

7. Verfahren nach einem der Ansprüche 3 bis 6, **dadurch gekennzeichnet, daß** der unterste Boden gegenüber der üblichen Position in der Kolonne um das bis zu 6-fache des Abstandes der Böden in der Kolonne zueinander höher angeordnet ist.

8. Verfahren nach einem der Ansprüche 3 bis 7, **dadurch gekennzeichnet, dass** der unterste Boden gegenüber der üblichen Position in der Kolonne um das 2- bis 4-fache des Abstandes der Böden in der Kolonne zueinander höher angeordnet ist.

9. Verfahren nach einem der Ansprüche 3 bis 8, **dadurch gekennzeichnet, dass** der unterste Boden gegenüber der üblichen Position in der Kolonne um das 3-fache des Abstandes der Böden in der Kolonne zueinander höher angeordnet ist.

10. Verfahren nach einem der Ansprüche 3 bis 6, **dadurch gekennzeichnet, daß** zumindest die untersten 1 bis 6 Böden als Dual-Flow-Böden ausgebildet sind.

11. Verfahren nach einem der Ansprüche 3 bis 6, **dadurch gekennzeichnet, dass** zumindest die untersten 2 bis 4 Böden als Dual-Flow-Böden ausgebildet sind.

12. Verfahren nach einem der Ansprüche 3 bis 6, **dadurch gekennzeichnet, dass** zumindest die untersten 3 Böden als Dual-Flow-Böden ausgebildet sind.

13. Verfahren nach Anspruch 10, **dadurch gekennzeichnet, daß** zumindest die Böden unterhalb des Seitenabzugs als Dual-Flow-Böden ausgebildet sind.

14. Verfahren nach einem der Ansprüche 1 bis 13, **dadurch gekennzeichnet, daß** als Sumpfverdampfer für die Destillationskolonne ein Fallfilmverdampfer eingesetzt wird.

## Claims

1. A process for the preparation of on-spec phthalic anhydride by distillative purification of crude phthalic anhydride, wherein crude phthalic anhydride is fed to a distillation column which is operated at reduced pressure, the low boilers are removed at the top or in the vicinity of the top of the distillation column and the on-spec phthalic anhydride is removed from the column via a side take-off.

2. A process as claimed in claim 1, wherein the distillation is carried out at a pressure at the top of the column of from 0.05 to 0.5 bar.

3. A process as claimed in claim 1 or 2, wherein the distillation is carried out in a tray column.

4. A process as claimed in claim 3, wherein the tray below and the tray above the point at which the side take-off is located are a distance apart which is up to 5 times greater than the usual distance between the trays in the column.

5. A process as claimed in claim 4, wherein the side take-off is located closer to the upper of the two trays.

6. A process as claimed in any of claims 1 to 5, wherein the on-spec phthalic anhydride is removed from the column in gaseous form via a side take-off.

7. A process as claimed in any of claims 3 to 6, wherein the lowermost tray is arranged higher by an amount of up to 6 times the distance between the trays in the column, compared with the usual position in the column.

8. A process as claimed in any of claims 3 to 7, wherein the lowermost tray is arranged higher by an amount of 2 to 4 times the distance between the trays in the column, compared with the usual position in the column.

9. A process as claimed in any of claims 3 to 8, wherein the lowermost tray is arranged higher by an amount of 3 times the distance between the trays in the column, compared with the usual position in the column.

10. A process as claimed in any of claims 3 to 6, wherein at least the lowermost 1 to 6 trays are in the form of dual-flow trays.

11. A process as claimed in any of claims 3 to 6, wherein at least the lowermost 2 to 4 trays are in the form of dual-flow trays.

12. A process as claimed in any of claims 3 to 6, wherein at least the lowermost 3 trays are in the form of dual-flow trays.

13. A process as claimed in claim 10, wherein at least the trays below the side take-off are in the form of dual-flow trays.

14. A process as claimed in any of claims 1 to 13, wherein a falling-film evaporator is used as the bottom evaporator for the distillation column.

## Revendications

1. Procédé de préparation d'anhydride phtalique conforme aux spécifications par purification par distillation d'anhydride phtalique brut, **caractérisé en ce qu'**on amène de l'anhydride phtalique brut à une colonne de distillation qui est mise en service sous pression réduite, on prélève les substances à bas point d'ébullition à la tête ou à proximité de la tête de la colonne de distillation et on prélève de la colonne l'anhydride phtalique conforme aux spécifications par un soutirage latéral.

2. Procédé suivant la revendication 1, **caractérisé en ce qu'**on effectue la distillation à une pression en tête de colonne de 0,05 à 0,5 bar.

3. Procédé suivant la revendication 1 ou 2, **caractérisé en ce qu'**on effectue la distillation dans une colonne à plateaux.

4. Procédé suivant la revendication 3, **caractérisé en ce que** le plateau situé en dessous de l'endroit où se trouve le soutirage latéral et le plateau situé au-dessus de cet endroit présentent entre eux une distance agrandie jusqu'à cinq fois par rapport à la distance courante entre les plateaux dans la colonne.

5. Procédé suivant la revendication 4, **caractérisé en ce que** le soutirage latéral se trouve plus près de celui des deux plateaux qui est supérieur.

6. Procédé suivant les revendications 1 à 5, **caractérisé en ce que**, par un soutirage latéral, on prélève l'anhydride phtalique conforme aux spécifications de la colonne sous une forme gazeuse.

7. Procédé suivant l'une des revendications 3 à 6, **caractérisé en ce que** le plateau inférieur est, par rapport à la position habituelle dans la colonne, agencé plus haut de jusqu'à 6 fois la distance entre les plateaux dans la colonne.

8. Procédé suivant l'une des revendications 3 à 7, **caractérisé en ce que** le plateau inférieur est, par rapport à la position habituelle dans la colonne, agencé plus haut de 2 à 4 fois la distance entre les plateaux dans la colonne.

9. Procédé suivant l'une des revendications 3 à 8, **caractérisé en ce que** le plateau inférieur est, par rapport à la position habituelle dans la colonne, agencé plus haut de 3 fois la distance entre les plateaux dans la colonne.

10. Procédé suivant l'une des revendications 3 à 6, **caractérisé en ce qu'**au moins les 1 à 6 plateaux inférieurs sont réalisés sous la forme de plateaux à double écoulement.

11. Procédé suivant l'une des revendications 3 à 6, **caractérisé en ce qu'**au moins les 2 à 4 plateaux inférieurs sont réalisés sous la forme de plateaux à double écoulement.

12. Procédé suivant l'une des revendications 3 à 6, **caractérisé en ce qu'**au moins les 3 plateaux inférieurs sont réalisés sous la forme de plateaux à double écoulement.

13. Procédé suivant la revendication 10, **caractérisé en ce que** les plateaux situés en dessous du soutirage latéral sont réalisés sous la forme de plateaux à double écoulement.

14. Procédé suivant l'une des revendications 1 à 13, **caractérisé en ce que**, comme évaporateur de fond de la colonne de distillation, on met en oeuvre un évaporateur à film tombant.
